# EUROPEAN PATENT APPLICATION

(11) **EP 4 495 595 A1**
(43) Date of publication of application: **22.01.2025**
(21) Application number: 24187125.0
(22) Date of filing: 08.07.2024
(51) Int. Cl.: G01N 33/00, G01N 1/22, G01N 33/24

(54) **MULTI-SENSOR DEVICE OF MODULAR DESIGN FOR THE DETECTION OF NATURAL HYDROGEN DEPOSITS AND HELIUM AND METHOD FOR DETECTING DEPOSITS OF NATURAL HYDROGEN AND/OR HELIUM**

(30) Priority: 13.07.2023 PL 44557823
(71) Applicant: Solar System Resources Corporation Sp z o.o, 31-153 Kraków (PL)
(72) Inventor: Zwierzynski, Adam, 31-303 Cracow (PL); Boron, Piotr, 32-340 Wolbrom (PL)
(74) Representative: Kondrat, Mariusz

(57) **Abstract**

The object of the invention is a system for detecting natural deposits of hydrogen and helium, in particular deposits of natural hydrogen also known as "golden hydrogen" or "white hydrogen", and locating potential geological sites where deposits of so-called "white hydrogen" can be produced by stimulation with water 'orange hydrogen'. The system aggregates and processes terrain mapping data (optical, SAR, gravimetric and geomagnetic data from satellite, aerial or drone imagery) with weather and ground sensor data.

## Description

The subject of the invention is a multi-sensory device of modular design for the detection of deposits of natural hydrogen and helium, in particular deposits of natural hydrogen also called "golden hydrogen" or "white hydrogen" and the location of potential geological sites where deposits of so-called "orange hydrogen" can be produced by stimulation with water. The system aggregates and processes terrain mapping data (optical, SAR, gravimetric and geomagnetic from satellite, aerial, or drone imagery) with weather and ground sensor data.

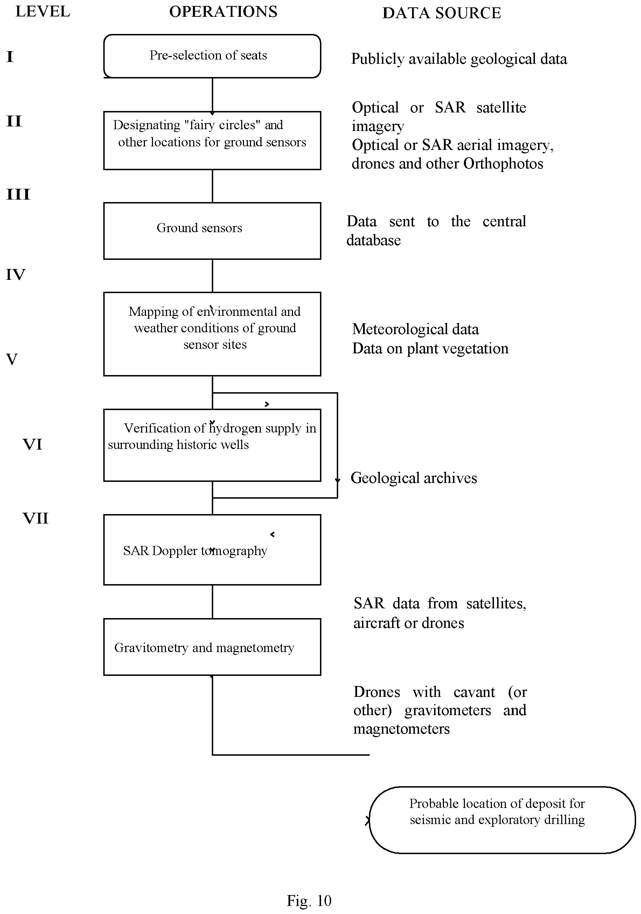

An integral part of the system for detecting natural hydrogen and helium deposits is the multi-sensor device for monitoring the composition of ground gases described in the patent application and the algorithm shown in Fig. 10 also described in the patent application. The innovativeness of the system and its components, including the aforementioned algorithm, is inextricably linked to the type of application for which it is to be used, i.e. the search for deposits of natural hydrogen and helium, whose industrial exploration in the world is only at starting point.

Sensors measuring the concentration of gases such as hydrogen, helium and radon have been known for decades. One of the significant novelties of the presented invention is that they are used in combination with other data (terrain imaging, weather data) to create a system for detecting potential sites of natural hydrogen, which is not an obvious solution and required the development of a novel algorithm. It should be emphasised that until recently there was little scientific evidence of natural hydrogen deposits worldwide, let alone in Europe. It is also not obvious what combination of gas sensors should be used to effectively detect potential natural hydrogen deposits. Relying solely on a sensor that measures hydrogen concentration levels in the ground can be highly misleading, as diurnal hydrogen inputs fluctuate and concentration levels are influenced by, for example, decay processes in the soil. Monitoring should also be carried out over a period of at least several months to exclude temporary increased hydrogen inputs. In order to be able to obtain unambiguous results, it is necessary to set up a system consisting of a network of sensors in which several types of gases - indicators - are measured and the data are reported to a central IT system and compared with weather data. It is also not obvious where such sensors should be deployed - data (optical, SAR, gravimetric and geomagnetic from satellite, aerial or drone imagery) can help with this. The object of the invention is therefore a complex multi-level system that serves to detect potential locations of natural hydrogen.

The solution presented is important for building a zero-carbon economy and a society of sustainable prosperity. The worsening climate crisis makes it urgent for humanity to seek new alternative energy sources. Economic factors, such as the rising price of emission quotas, are also adding to this, making the economies of countries relying on carbon-intensive technologies increasingly uncompetitive, with rising electricity prices in these countries being paid for by their populations. Poland faced an urgent need to accelerate its energy transition.

Renewable energy sources (RES) are a certain solution. However, their disadvantage is the high variability of daily production. Photovoltaic panels only produce electricity during the day, while households use most of it during the evening and night hours. With wind farms, on the other hand, there are times when the wind does not blow or is too strong and electricity cannot be produced. In order to be able to base power generation on RES, it is necessary to stabilise the power grid through energy storage or additional energy sources (such as nuclear power). In addition, extreme weather events can collapse the RES energy supply chain, as happened in February 2021 in Germany and Texas (USA), when snowstorms immobilised windmills and iced photovoltaic panels leading to an energy blockout and the sudden gigantic increase in electricity prices in Texas, and the power system had to be rescued using high-carbon electricity sources.

A solution could be the storage of surplus energy produced by RES. However, this is economically unviable for the time being. According to expert calculations, California would need more than 200 megawatt hours (MWh) of storage for every MW of wind and solar energy installed. Germany would probably manage with 150 MWh per MW. The current cost of battery storage (July 2023) is around US$600,000 per MWh. This means that for every MW of wind or solar power in California, $120 million would have to be spent on storage. In Germany, this would be $90 million.

A solution to cheaply store energy from RES could be to produce hydrogen using a water electrolysis process, store the hydrogen and then recover the electricity from hydrogen fuel cells. The problem is the high energy cost of the electrolysis process - 47 kWh of electricity must be consumed to produce 1 kg of hydrogen. Work is being done on catalysts to improve this process energetically, but so far this problem has not been solved. There are also no highly efficient methods on the market for the cheap storage and transport of hydrogen on an industrial scale. Another problem is the access to water sources in many locations where RES are located.

Hydrogen has many more potential applications than just energy storage. It can be used in metallurgy, heating, to power electric vehicles. This is the reason why it is considered one of the fuels of the future. Due to the way hydrogen is produced, there are several types of hydrogen:
- green hydrogen - the most desirable hydrogen for energy applications; it is obtained by electrolysis of water using energy from renewable sources, i.e. a source with no CO2 emissions. It is a green hydrogen.
- purple hydrogen - hydrogen obtained by electrolysis, but in power stations nuclear
- turquoise hydrogen - hydrogen obtained by pyrolysis of methane or by processing waste plastics. The pyrolysis process is carried out in an oxygen-free atmosphere with low CO2 emissions.
- Blue hydrogen - hydrogen produced from fossil fuels combined with CO2 capture processes
- grey hydrogen - hydrogen derived from steam reforming of natural gas or coal gasification with release of CO2 to the environment

Only green hydrogen is fully green. However, its price (July 2023) ranges from 3 to 8 euro/kg and there are the previously described problems with its production. Grey hydrogen, on the other hand, costs around 1.2 euro/kg (July 2023), but its production involves CO2 emissions into the atmosphere. The holy grail of green energy is cheap and environmentally friendly hydrogen. This problem can be solved by deposits of natural hydrogen also known as golden hydrogen or white hydrogen. This is a new concept, as until recently there was little scientific evidence that such deposits could occur in nature, despite the fact that hydrogen inflows have been recorded in various oil and gas wells in the past.

A failed well was drilled in Mali in 1987. Re-analysis of this well in 2012 showed that it produced 98% hydrogen. This was one of the world's first documented hydrogen deposits, which was later developed - the hydrogen powers a fuel cell that generates electricity for the surrounding village. Previously, scientists had not considered that there might be natural hydrogen deposits on Earth. Since 2018, when the Malian field was described in the International Journal of Hydrogen Energy, the number of articles on natural hydrogen has started to increase. At the end of 2022, more discoveries of natural **hydrogen** began to appear in the world literature. Deposits of natural hydrogen were discovered in Australia, which became the first country in the world to launch natural hydrogen exploration licences. In April 2023, Helios Aragon (a subsidiary of BP - British Petroleum) announced the discovery of a deposit of natural hydrogen (and helium 4%) on the reconnaissance area near Monzón and Barbastro in the Aragon region of Spain. This is the first documented discovery of hydrogen and helium deposits in Europe. The initial estimate of the deposit is 14.16 billion m3. In June 2023, it was reported in the media that La Française d'Energie (FDE) had discovered a large deposit of natural hydrogen in the Lorraine basin, which could contain 46 million tonnes of natural hydrogen, equivalent to half of the world's current hydrogen production and enough to make a significant contribution to the EU's decarbonisation targets. There is speculation that deposits of natural hydrogen may also exist in Poland.

In a wave of reports about the first detected deposits of natural hydrogen, the first companies offering dedicated ground sensors to monitor hydrogen concentrations and thus detect anomalously high hydrogen flows, which could be a signal that a deposit of natural hydrogen is located in the area.

Australian company Axiom Sensing has developed the WHALI (White Hydrogen Autonomous Logging Instrument) solution, which contains four gas sensors (H2, CO2, CH4, H2S), temperature and atmospheric pressure sensors. The instrument is powered by a small photovoltaic panel. The probe is driven 1 metre deep into the ground and the gases from the probe are extracted by a small pump and analysed at the surface. The WHALI has a 3G/4G internet module to provide connectivity to a mobile network base station (or satellite) for data transfer to the control room. WHALI also incorporates a water management system to take measurements in areas with high water levels. The WHALI takes the form of a small fenced measuring station.

The disadvantage of the WHALI solution is that it is tailored to the conditions of Australia, where there is vast wilderness in the centre of the continent, but it is less likely to work in European conditions, where there is high population density and a lot of agricultural land. Agricultural landowners may not wish to have measurement stations installed on their land that would protrude above the ground and impede the passage of a farm tractor and, in particular, significantly limit the ability of the farm tractor to operate in autonomous mode based on GPS navigation. The WHALI solution makes it possible to measure the concentration of several types of gas in the soil gas, but the measurement is made at the surface and the sensors are not placed inside the borehole.

Another solution that has emerged worldwide is the result of the SurfMog H₂ project carried out by a consortium comprising the French company 45-8 ENERGY and the Swiss company SOLEXPERTS. This solution does not have the described disadvantages of the WHALI solution, as it is installed entirely in the drilled hole, with only a flat communication system protruding on the surface. Using a hand drill, a hole is drilled into which a casing pipe is mounted in which the device is placed. The device has a diameter of 7 cm and a length of 100 cm. At the bottom of the device is the hydrogen sensor, above it is the battery system and logger, and on the surface is the flat telecommunications system, which connects to a mobile network base station to transmit data to a central station. The unit can be easily pulled out of the casing pipe to replace the hydrogen sensor with a CH4 or CO2 sensor. The disadvantage of the solution is that it only measures the level of one ground gas. The sampling time is 30 minutes (2 measurements per hour) and the operating time is 1 year with data transmission once a day.

The essence of the invention is a multi-sensor device of modular construction for the detection of natural hydrogen and helium deposits, constructed from a casing tube on the sides of which electrodes are placed and a protective disc characterised by the fact that in the casing tube there is a spacer on which the gas sensor module is placed, followed by a radon sensor module, a pumping module, a battery module, a logger and a transmitter/receiver system located in the valve head, the casing tube is equipped with an inlet inserted into the grooves and running inside the casing tube 1 along its entire length, inside which a probe measuring the soil pH is placed, which protrudes beyond the casing tube.

Preferably, the protective disc takes the form of a metal mesh with a mesh size of < 50 micrometres.

Preferably, the spacer has eight legs which form, at the base of the element, eight chambers connected by an annular groove crossing each leg, each chamber contains a single ground gas inlet channel which leads to a single sensor for the detection of a specific gas type, the sensor being located in the gas sensor module, in addition to which there is a water channel in a groove in one of the legs.

Preferably, each inlet opening of the ground gas duct is surrounded by a socket in which the protective ring with a metal mesh size of less than 50 micrometres is fitted.

Preferably, in the central part of the spacer there is a radon inlet channel which runs inside a cylinder protruding from the spacer and which leads to a radon sensor located in the radon sensor module, the inlet of the radon inlet channel being surrounded by a socket in which a protective ring with a metal safety net is mounted while the cylinder passes through the gas sensor module and leads to the radon sensor module.

Preferably, a ring with an internal thread protrudes in the upper part of the spacer, to which the gas sensor module is screwed.

Preferably, in the central part of the gas sensor module there is a through circular opening through which a cylinder belonging to the spacer passes and enters the radon sensor module, the gas sensor module being internally divided into eight separate isolated chambers, in each of which a single sensor measuring the concentration of a gas selected from hydrogen, helium, nitrogen, CO2, CH4, H2S is placed and the measurement data from each sensor is collected by an internal electronic circuit and transmitted to the logger via a bus.

Preferably, a separate opening leads to each of the eight chambers in the gas sensor module, which is an extension of the inlet channels of the soil gas, which are located in the spacer, and from each of the eight chambers in the gas sensor module, the analysed gas is discharged through a separate outlet channel; whereby a separate and insulated channel also leads through the gas sensor module, through which water pumped from the bottom of the hole flows and the channel is an extension of the drainage channel in the spacer and leads to the pumping module.

Preferably, there are channels through the radon sensor module, which discharge gas from the chambers of the gas sensor module to a pumping module located above the radon sensor module, with an opening at the bottom of the radon sensor module and an opening at the top of the radon sensor module, with a radon sensor located in the radon sensor chamber.

Preferably, the radon sensor module is provided with two rings: an upper ring and a lower ring, which have female threads.

Preferably, in the lower part of the pumping module there are openings which feed respectively the gases from the chambers of the gas sensor module (5), the gas from the radon sensor module, the water pumped from the bottom of the interior of the lining pipe, moreover, a channel comes out of the pumping module while a threaded cylinder protrudes at the bottom, which enables its connection with the earlier radon sensor module.

Preferably, the battery module connects to the downstream pumping module and the downstream logger respectively by means of rings with external threads, furthermore a keyway running along the battery module is an extension of the keyways present in the elements respectively.

Preferably, on the surface of the casing pipe there is a valve head with a transmitter-receiver system in the form of a spherical bowl, which is connected to the other modules by means of a ring with an internal thread, whereby there is an opening on the spherical bowl through which water, as well as ground gases, is removed in the form of a spray; in addition, there are also LEDs on the spherical bowl.

Another object of the invention is a method for detecting deposits of natural hydrogen and/or helium using a multi-sensor device of modular design characterised in that a combination of hydrogen and helium and/or radon detection is used, the method comprising the following steps:
a) preselection of sites on the basis of publicly available geological data;
b) search for so-called 'fairy eyes' using optical or radar imagery from satellites, airborne or drones, and orotophotos may also be used;
c) installation of the equipment according to claim. 1 at the sites selected in steps a) and b) for a period of at least several months, in order to study the daily changes in the composition of the soil gas on the basis of data periodically transmitted from the devices to a central database via a mobile network or, in the absence thereof, via satellite; in addition, information on the location of the device is transmitted, the determination of which is made possible by an integrated GPS receiver;
d) Mapping the environmental and weather conditions of equipment sites by checking the weather conditions in the area of the installation of a particular sensor, data on the humidity and pH of the ground, air temperature and pressure from the sensors in the equipment;
e) performing SAR Doppler tomography of a selected area;
f) performing a flight over the site selected in previous steps a) to e) by a drone or aircraft with a gravimetric sensor and a magnetic sensor installed;
g) identification of the likely location of natural hydrogen and/or helium deposits, where seismic geophysical surveys should be initiated and a test hole drilled.

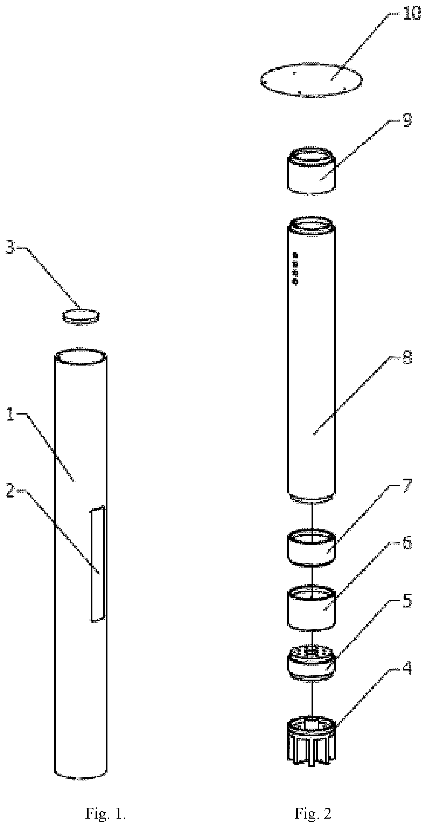

The developed solution 'HydroHelium Manifestor', which is the subject of the invention, is a complex system for the detection of natural hydrogen and helium deposits. One of the components of this system is a network of multi-sensor ground gas composition monitoring devices called HHM (the abbreviation of the system's name), which constitute one of the seven layers of the entire system. The HHM devices consist of a casing pipe 1 made of metal or plastic, as shown in Fig. 1. On opposite sides of the casing pipe 1, electrodes 2 are located, which are used for resistometric measurements of the soil, making it possible to determine the water content of the soil, as well as other soil parameters. Excessive soil moisture can lead to decay processes of vegetation and production of hydrogen of biotic origin (produced as a result of microbial activity), which will interfere with the measurement of hydrogen extracted from geological formations. At the bottom of the casing pipe 1 there is a protective disc 3 in the form of a metal mesh with microscopic meshes, which makes it difficult for water to enter the casing pipe, but does not impede the migration of ground gases, which can penetrate the microscopic meshes of the mesh more easily than water having a high surface tension.

Into the casing tube 1 is inserted the kit shown in Fig. 2, consisting of a spacer 4, a gas sensor module 5, a Radon-220 sensor module (optionally ²²⁰Rn and ²²²Rn sensors) 6, a pumping module 7, a battery module 8, a logger 9, a valve head which is a transmitter-receiver system 10. The proper orientation of the set of elements 4-10 introduced into the casing tube 1 is ensured by a groove running inside the casing pipe 1 through its entire length inside which the probe measuring the pH of the soil, protruding slightly outside this casing tube, is hidden.

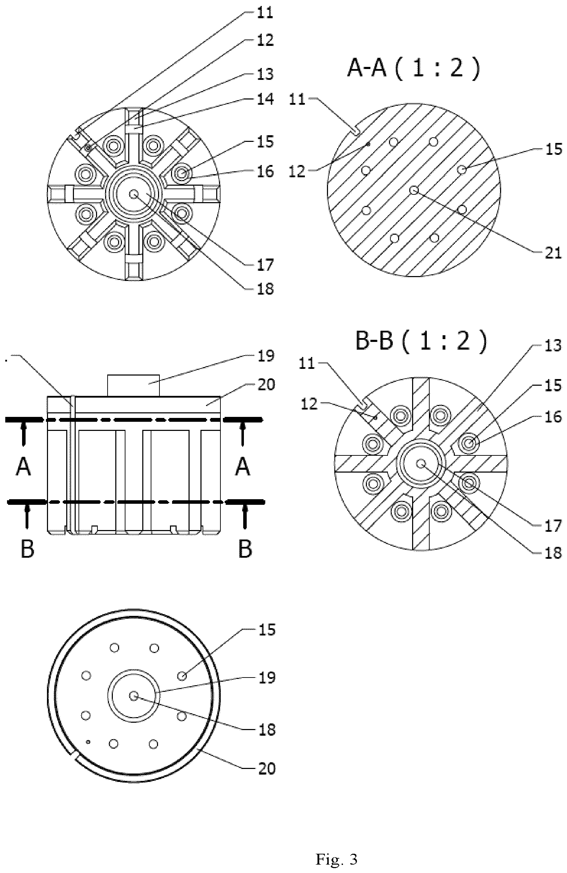

The spacer 4 is shown in simplified form in Fig. 3. The drawing on the top left shows the bottom view, while the drawing on the bottom left shows the top view. The spacer 4 has a keyway 11 running along its entire length on its side. An identical keyway is also present in the spacer 5, 6, 7 and 8 running along their entire length. The keyway 11 provides proper orientation of the assembly comprising elements 4 - 10 in the casing pipe 1. The spacer 4 has eight legs 13, which form eight spaces at the base of the spacer 4 connected by an annular groove 14 crossing each leg. Should water accumulate at the bottom of the casing pipe 1, the groove 14 will ensure that the water is evenly distributed. The groove 14 in one of the legs projects a guide channel 12, which enables the pumping module 7 to drain water from the bottom of the interior of the casing tube 1 without it entering the sensors. The purpose of the feet 13 is to separate the inlets to the channels leading the soil gas to the sensors from the bottom of the cladding tube, where water can accumulate, thus providing additional protection against water entering the sensors. The legs 13 divide the spacer 4 into eight interconnected chambers, each containing a single ground gas inlet channel 15, which leads to a single sensor detecting a specific type of gas, which sensor is located in the gas sensor module 5. Each channel inlet opening 15 is surrounded by a socket 16 in which a protective ring with a metal mesh is mounted to further protect against water in g res s . This mesh has microscopic meshes (even smaller than the protective disc 3 located at the bottom of the liner tube 1) through which gas can pass freely, but water cannot enter. In the centre of the spacer 4 there is a separate radon inlet channel 18, which leads independently to the radon sensor located in the radon sensor module 6. The inlet channel 18 is surrounded by a socket 17 in which a protective ring with a metal mesh is mounted to prevent water ingress. The radon inlet channel 18 runs inside a cylinder 19 protruding from the spacer 4. The cylinder 19 passes through the gas sensor module 5 and leads directly to the radon sensor module 6.

The design of spacer 4 prevents water from entering the sensors, enables the level of water accumulating at the bottom of the lining pipe to be monitored and pumped out, and enables the soil gas to be drawn independently into each of the gas sensors individually, which significantly increases measurement reliability and the reliability of the device. A ring 20 with an internal thread protrudes from spacer 4 at the top, to which the gas sensor module 5 is screwed.

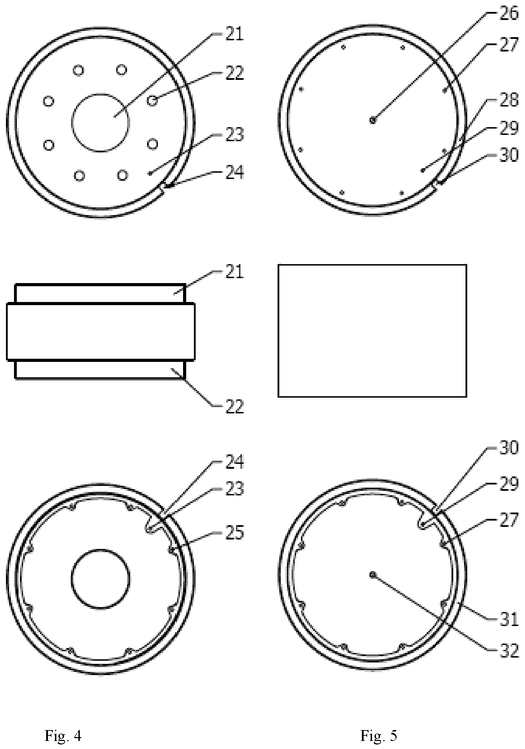

The gas sensor module 5 is shown in simplified form in Fig. 4. It is connected to the previous module (spacer 4) and the next module (radon sensor module 6) by means of rings 22 and 21 with external threads, respectively. The top figure shows a bottom view of element 5, the middle figure shows a side view of element 5 and the bottom figure shows a top view of element 5. In the centre of the gas sensor module 5 there is a through circular hole 21 through which (after assembling the elements 4 - 6) a cylinder 19 belonging to the spacer 4 passes and enters the radon sensor module 6. The gas sensor module 5 is internally divided into eight separate isolated chambers in each of which a single sensor measuring the concentration of a specific type of gas is placed. The data from each sensor is collected by an internal electronic circuit and transmitted to the logger 9 via a bus, which is not shown in Fig. 4 for simplicity, as are the internal and external seals. In the gas sensor module 5 the sensors are located:
- hydrogen sensor,
- helium sensor,
- nitrogen sensor,
- CO2 sensor,
- CH4 sensor,
- H2S sensor
- the other 2 sensors as required,

The two most relevant sensors are hydrogen and helium. A separate opening 22 leads to each of the eight chambers in the gas sensor module 5, which is an extension of the ground gas inlet channels 15, which are located in the spacer 4. Similarly, from each of the eight chambers in the gas sensor module 5, the analysed gas is discharged through a separate outlet channel 25. A separate and isolated channel 23 also leads through the gas sensor module 5, which carries water pumped from the bottom of the borehole. The channel 23 is an extension of the guide channel 12 in the spacer 4 and leads to the pumping module 7. At the shunt of the sensor module gas 5 there is a keyway 24, which is an extension of the keyway 11 in the element 4.

The radon sensor module 6 is shown in Fig. 5. Two variations of this module are possible - identifying and measuring the concentration of ²²⁰Rn isotope only or identifying and measuring the concentration of ²²²Rn and ²²⁰Rn isotopes. In Fig. 5, the top figure shows a bottom view of the radon sensor module 6, the middle figure shows a side view of the radon sensor module 6 and the bottom figure shows a top view of the radon sensor module 6. On the flank of the radon sensor module 6 there is a groove for groove 30, which is an extension of the groove for groove 24 in the gas sensor module 5 and the groove for groove 11 in the spacer 4. Through the radon sensor module 6 pass channels 27, which discharge gas from the chambers of the gas sensor module 5 to a pumping module 7 located above the radon sensor module 6. The gas streams flowing through channels 27 do not mix with the gas stream supplied to the radon sensor located inside the radon sensor module 6. Channel 29 carries a stream of water pumped from the bottom of the cladding tube and is isolated. Channels 27 and 29 are extensions of channels 25 and 23, respectively, found in the gas sensor module 5. Ground gas for analysis is fed to the radon sensor module 6 via hole 26 (which is an extension of the axis of channel 18 found in spacer 4) and discharged via hole 32. As the name suggests, inside the radon sensor module there is an isolated chamber in which a sensor for detecting the presence and measuring the concentration of radon is placed. The radon sensor module 6 has two rings: an upper ring 31 and a lower ring 28, which have internal threads and are used to assemble this module into a set with other modules (gas sensor module 6 below and pumping module 7 above).

The monitoring of multiple gas indicators represents a major innovation in the search for natural hydrogen and helium deposits. While there are solutions in the world that monitor the concentration of several types of gas in ground gas to detect hydrogen, helium and radon have not previously been used for this purpose. Hydrogen surges do not necessarily have to originate from the hydrogen reservoir, but could be the result of decay or other biotic processes. If the hydrogen in the deposit came from its release from the Earth's mantle then it is highly likely that helium was also released along with it. Both gases are found in the Earth's mantle and were accumulated there during the formation stage of our planet. Scientists studying the topic of natural hydrogen were surprised to find that helium is also often found in deposits of natural hydrogen. This is because hydrogen molecules and helium atoms have similar migration properties in different rock media. The van der Waals radius of a hydrogen molecule is 120 pm, while that of a helium atom is 140 pm. In contrast, the kinetic radius (a measure relating to atoms and molecules that determines the probability that a molecule in a gas will collide with another molecule) is 289 pm for a hydrogen molecule and only 260 pm for helium, meaning that helium will migrate slightly faster than hydrogen molecules. Therefore, natural hydrogen deposits are often accompanied by helium. If, therefore, the inflowing hydrogen will have an admixture of helium then it can be presumed that it originates from natural geological formations and is not the result of microbial activity and therefore of biotic origin. Accumulated natural hydrogen may, however, originate from serpentinisation processes in the rocks and may also be the result of radiolysis of water in radioactive formations. In such cases, it is likely that helium will not always accompany the hydrogen. Seismologists have long noted that radon can be an indicator of the possibility of earthquakes - the accumulating stresses in the Earth's crust cause the formation of microcracks through which radioactive radon is released to the surface. The natural hydrogen that comes to the surface comes from a deposit that may be remote and migrates to the surface also through microcracks. Therefore, if radioactive radon is detected in the hydrogen it is a signature that it may be coming from within the earth. In particular, the concentration of the isotope ²²⁰Rn which has a long half-life, should be monitored, ensuring that it originates from within the Earth rather than being the result of atmospheric phenomena that can lead to a temporary accumulation of the isotope ²²²Rn (thoron). The occurrence of the isotopes ²²⁰Rn (T1/2 = 3.82 days) and ²²²Rn (T1/2 = 56 seconds) in the inflowing hydrogen may be a strong indicator that it originates from within the Earth. The publication [4] also indicated a correlation of nitrogen occurrence in helium deposits. Therefore, measuring the nitrogen concentration in the inflowing helium seems highly desirable, although the method of using nitrogen as an indicator of helium deposits still needs to be refined in particular that nitrogen in ground gas can also come from fertilisers.

**The monitoring of multiple gas indicators in particular in the system (H₂/He/²²²Rn/N₂) or (H₂/He/²²²Rn/²²⁰Rn/N₂)** represents a major innovation in the worldwide search for natural hydrogen and helium deposits, which has been applied in the device that is part of the system for the search for natural hydrogen and helium deposits that is the subject of the patent application. Placing these sensors inside the borehole, close to the bottom of the borehole, is another innovation that increases the precision of the measurements - hydrogen is a highly permeable gas, pumping it out to the measuring equipment at the surface increases the risk that unwanted hydrogen from other processes in the environment (e.g. industrial processes) not originating from ground gases may enter the measuring system, which may give false readings. This is particularly relevant if there is little natural hydrogen input from its reservoir due to the local geological structure.

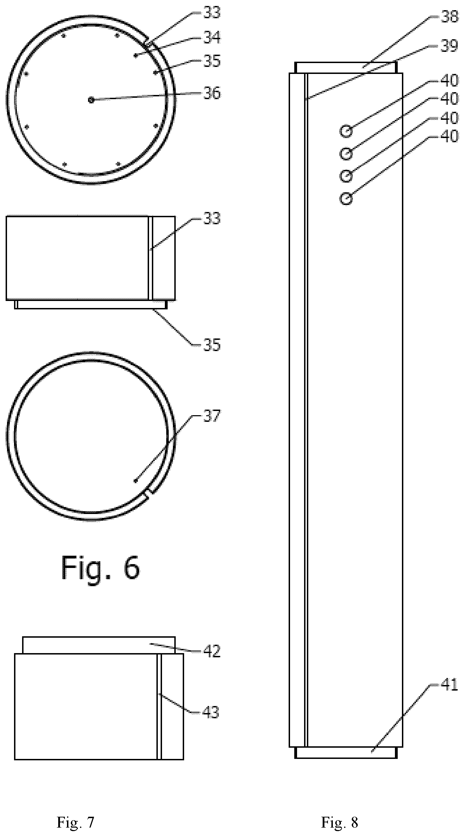

Fig. 6 shows the pumping module 7. As in the preceding figures, the top figure shows a bottom view of the pumping module 7, the middle figure shows a side view of the pumping module 7 and the bottom figure shows a top view of the pumping module 7. On the side view of the pumping module 7 is located the groove for the groove 33, which is an extension of the grooves for the grooves for the grooves 30, 24, 11 present in the elements 6, 5, 4, respectively. In the lower part of the pumping module 7 are located the openings 35, 36, 34, which supply respectively the gases from the chambers of the gas sensor module 5; the gas from the radon sensor module 6; the water pumped from the bottom of the interior of the cladding tube 1. From the pumping module 7, the water and the ground gas are discharged to the surface by means of a channel 37. From the pumping module 7, a threaded cylinder 35 protrudes at the bottom, which enables its connection with the former radon sensor module 6.

The developed device, which is part of the system that is the subject of the patent application, is characterised by the fact that a separate channel leads from each of its sensors to the pumping module, and the water pumped from the bottom of the borehole is also led through a separate isolated channel. This allows the pumping module 7 to operate in as many as twelve modes:
- Simultaneous pump-out mode for water and gases from all channels;
- Pump-out mode - only water from the bottom of the pipe is removed lining, but no soil gas is drawn into the sensor chambers;
- Ground gas aspiration mode for all sensor chambers;
- 8 aspiration modes in which ground gas is aspirated exclusively into one of a further eight chambers (containing a sensor measuring only one type of gas) in the gas sensor module 5;
- Suction mode in which ground gas is sucked exclusively into the radon sensor module 6.

As most of the inlet channels to pump module 7 are circular, a rotary element in pump module 7 is used to switch between the various modes, which, depending on its angle of rotation, blinds the corresponding channels. The introduction of so many (as many as twelve) operating modes in pump module 7 is a significant improvement to facilitate the search for natural hydrogen and helium deposits. Sensors for different types of gas can have different sensitivities. This means that in order to detect two different types of gas with the sensors measuring them, it may be necessary to pass different volumes of analysed ground gas through them. Flexible management of the pumping capacity of the pumping module 7, combined with the twelve operating modes of this module, gives much greater possibilities for detecting changes in composition, saving energy and using different sensor types with different sensitivities.

Fig. 7 shows the battery module 8. It is connected to the preceding pumping module 7 and the following logger respectively by means of rings 41 and 38 with external threads. Throughout the entire length of the battery module runs a keyway 39 which is an extension of the keyways 33, 30, 24, 11 present in the elements 7, 6, 5, 4 respectively. These grooves ensure the proper orientation of the set of elements 4-10 inserted into the casing tube 1, so that the contacts of the 40 occurring on the battery module are connected to internal contacts in the casing pipe, allowing resistivity measurements of the soil (thanks to electrodes 2 located on the outer side of the casing pipe 1) and pH measurements of the soil (thanks to a probe hidden in a groove running inside the casing pipe 1 through its entire length, into which grooves fit under the keyway 33, 30, 24, 11 found in elements 7, 6, 5, 4 respectively). The battery module 8 is the longest element of the set 4 - 10 inserted into the casing pipe 1 and contains a set of replaceable batteries/batteries, manages the energy, manages the measurements including their recording in the logger 9 once transmitted to the control panel.

Logger 9, shown in Fig. 8, is for data storage only and is placed close to the surface so that, in the event of a failure of the transceiver system, service personnel could easily pull it out and retrieve the data. It is a type of flash memory. Like the other components, it contains a keyway 43 and a protruding threaded cylinder 42.

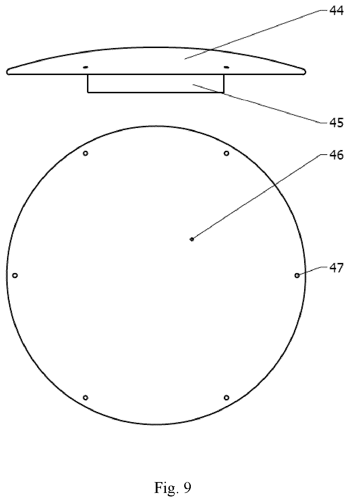

Figure Fig. 9 shows a valve head with a transmitting and receiving system 10 on the surface. A spherical bowl 44 protrudes minimally above the ground surface and is connected to the other components of the set 4 - 9 by means of a ring with an internal thread 45. On the spherical bowl, there is an opening 46 (with a nozzle) through which water and also ground gases are removed in the form of spray. The removal of water in spray form ensures that it does not flood the casing pipe again - e.g. water particles can be carried away by the wind. There are also LED 47s on the spherical canopy, which flash a weak light to make it easier to find the device at night or with flying drones. The valve head with Transceiver System 10 includes a module that connects to the cellular network and a satellite module that is activated when the devices are located in areas outside of cellular network coverage. It also contains a GPS module that allows the location of the device to be determined. The valve head with transceiver system 10 also contains a temperature and air pressure sensor.

In addition, the valve head with transceiver system 10 contains a recantenna that allows the device to be wirelessly charged by a microwave beam directed at it, e.g. from a Space Based Solar Power (SBSP) system. The small surface area of the recantenna is compensated for by the small current required to fully charge the battery module 8 and the long charging time. Although there are currently no SBSP (Space Based Solar Power) orbital energy stations in the world to deliver energy on an industrial scale, work is underway to develop such systems. Such systems will be able to send energy thousands of kilometres away with the flexibility to redirect it where it is needed. A study by Frazer-Nash Consultancy for the UK government in 2022 demonstrated the technical feasibility and cost-effectiveness of such a project. Alongside the development of fusion power, the construction of SBSP orbital energy stations has been embedded in the UK's development strategy. The Space Energy Initiative has been established to develop the UK's SBSP system. The European Space Agency (ESA) is also working on its S B S P system, having published feasibility studies commissioned by it for the SOLARIS Programme in August 2022. Although the first SBSP systems will be developed in 10 years' time, SBSP technology demonstrators are currently being built and one European company also has plans to develop a system to power various devices using guided microwave beams. The device being developed as part of the system that is the subject of the patent application, thanks to the possibility of additional optional wireless power supply by means of a guided microwave beam, will be able to operate for decades with a reduced number of service operations related to battery replacement or charging. This will also allow the devices to be placed in the middle of nowhere where human service operations are not possible. With this additional wireless charging function, the device can also serve as a measuring station to provide data for correcting the microwave beam in relation to ionospheric disturbances by measuring the intensity of the microwave beam against a theoretical value.

An integral part of the system subject of the invention is the algorithm shown in Fig. 10, which defines the seven information-technical levels/layers of the system. The algorithm is an integral part of the operating principle of the described multi-sensor HHM device monitoring HHM ground gases and the system based on a network of HHM devices.The first step of the algorithm is to preselect sites based on publicly available geological data. An analysis of the general geological conditions can pre-select regions that may be suspected of having geological conditions conducive to the formation of natural hydrogen and helium deposits. This step makes it possible to narrow down the search area and thus reduce the IT resources consumed and the associated costs. This is the first layer of the system, which can be omitted in some applications.

The second step of the algorithm is to search for so-called 'fairy meshes'. These are circles distinguished by the fact that there is no vegetation in the area. They are shaped like circles, usually concave, and some of them may be flooded with water. There are various theories to explain the appearance of such formations in the terrain. One is that they are sites of natural hydrogen escape. To detect "fairy meshes" method uses optical or radar imagery from satellites, airborne or drone imagery, and orotofotomaps can also be used. Radar data (especially SAR) can also provide information on the changing dielectric properties of the ground, which can also be helpful in selecting sites of interest for further analysis. This is the second layer of the system.

The third step is to install multi-sensor HHM ground gas monitoring devices at selected (in steps I and II) sites for a period of at least several months to study daily changes in ground gas composition. Data from the HHM devices are periodically transmitted to a central database via a mobile network or, in the absence of a mobile network, via satellite. Information on the location of the HHM device is also transmitted, which is determined by a built-in GPS receiver. This is the third layer of the system.

HHM devices can show hydrogen influx, but this does not yet mean that it is of abiotic origin (i.e. not the result of microbial activity) and comes from a natural hydrogen deposit. Admittedly, the analysis of multiple indicators can give a clear answer, but there may be geological cases in which only hydrogen flows in. Therefore, the fourth step is to map the environmental and weather conditions of the HHM sites. The weather conditions in the area where a particular HHM sensor is installed are checked - for example, the occurrence of heavy rainfall may mean that the elevated hydrogen readings occurring later were from septic processes. Data on soil moisture and pH, air temperature and pressure from the sensors in the HHM unit are checked. From optical satellite imagery, the vegetation parameters of the area are determined, and (if multispectral and hyperspectral imagery is available) whether the area has been fertilised (which can cause high nitrogen readings) or whether there have been industrial processes in the area that may have affected the measurement. This step helps to eliminate false data. This is the fourth layer of the system.

The fifth step is to verify that there have been no hydrogen or helium inflows in the surrounding historical wells (within a 50 km radius of the location of the HHM device with a positive indication) in the past. For example, in Poland (as of 2020), the Central Geological Database (CBDG) contains information on almost 218 000 boreholes, of which approximately 24 000 are more than 200 m deep. On the other hand, the Database of Geological and Engineering Data (BDGI) contains information on almost 328 000 (as of 2020) geological and engineering boreholes. Both databases are maintained as part of PSG's tasks. For some of the boreholes, inflow surveys were performed and data on these surveys are available. If the indications of HHM equipment located in an area are positive and anomalous inflows of hydrogen or helium have been recorded in the surrounding (even historic) borehole, this is a strong indication that a deposit of natural hydrogen or helium should exist somewhere in the area. This is the fifth layer of the system. As the aforementioned borehole data may not always be available sometimes this step will be omitted.

The sixth step is to perform SAR Doppler tomography of the selected terrane. The presence of anomalous hydrogen or helium inflows in an area does not mean that a natural hydrogen or helium deposit is present. This deposit may be located elsewhere, and the gas from the deposit may migrate many kilometres through fractures and microfractures present in the Earth's crust. The geological structure of the selected sites should be analysed. On the other hand, it is too early in the verification phase to use classic expensive seismic surveys. For this purpose, the innovative Doppler tomography technology will be used.

SAR radar - an example of one version of such technology is described in a patent application registered with the Malta Patent Office (Commerce Department - Industrial Property Registrations Directorate) under number PT4451, entitled 'Synthetic aperture radar underground, undersea, underice, and inside distributed targets tomographic doppler imaging'. SAR radar data can come from satellite or drone imaging (alternative sensors to SAR that give radar imaging can also be used). Naturally occurring or human-generated vibrations in nature cause small ground motions associated with vibrations of the induced rock mass. These cause Doppler shifts in the reflected radar signal. From these shifts, information about the geological structure of the radar-imaged terrain can be extracted up to many kilometres into the terrain. SAR Doppler tomography methods are particularly suitable for imaging voids, which can give information on whether the actual reservoir is located in the area, or whether gas is migrating through fractures and cracks from a reservoir located elsewhere. This is the sixth layer of the system.

The seventh step is to fly a drone or aircraft with a gravimetric sensor (preferably a quantum gravimeter) and a magnetic sensor installed over the site selected in the previous steps. Magnetic anomalies detected can provide key information on the nature of the mantle rocks, as magnetic susceptibility can be correlated with the degree of serpentinisation. Ultramafic rocks, such as those forming the lithospheric mantle, acquire their paramagnetism during serpentinisation reactions and the associated formation of magnetite. Gravimetric data, on the other hand, can provide information that rocks of a certain density are present beneath a given area. This further allows the sites selected in the previous steps to be verified. This is the seventh layer of the system.

The presented algorithm makes it possible to determine where natural hydrogen deposits are likely to occur, where geophysical surveys by seismic methods should be initiated and a survey hole drilled. By using the HydroHelium Manifestor system, the probability of detecting natural hydrogen and helium deposits can be dramatically increased and the costs of classical resource exploration for natural hydrogen deposits can be significantly reduced.

### Bibliography:

[1] Eric Hand, 'Hidden hydrogen. Does Earth hold vast stores of a renewable, carbon-free fuel?". Science, Vol 379, Issue 6633. https://www.science.org/content/article/hidden-hydrogen-earth-may-hold-vast-stores-renewable- carbon-free-fuel
[2] Qian-ning Tian, Shu-qing Yao, Ming-juan Shao, Wei Zhang, Hai-hua Wang, "Origin, discovery exploration and development status and prospect of global natural hydrogen under the background of carbon neutrality", China Geology, Vol. 5, Issue 4, October 2022, Pages 722-733 https://www.sciencedirect.com/science/article/pii/S2096519222002002#f3
[3] Lu Wang, Zhijun Jin, Xiao Chen, Yutong Su, Xiaowei Huang, "The Origin and Occurrence of Natural Hydrogen', Energies, Vol. 16, Issue 5 https://www.mdpi.com/1996-1073/16/5/2400
[4] Anran Cheng, Barbara Sherwood Lollar, Jon G. Gluyas & Chris J. Ballentine, "Primary N2-He gas field formation in intracratonic sedimentary basins," Nature Vol. 615, pages 94-99 (2023) https://www.nature.com/articles/s41586-022-05659-0
[5] Nicolas Lefeuvre, Laurent Truche, Frédéric-Victor Donzé,Maxime Ducoux, Guillaume Barré, Rose-Adeline Fakoury, Sylvain Calassou, Eric C. Gaucher, "Notive H2 Exploration in the Western Pyrenean Foothills", Geochemistry, Geophysics, Geosystems, Volume22, Issue 8, August 2021 https://agupubs.onlinelibrary.wiley.com/doi/10.1029/2021GC009917

### Supplementary bibliography on SBSP (Space Based Solar Power):

[6] Space Energy Initiative: https://spaceenergyinitiative.org.uk/
[7] Feasibility studies of the SOLARIS programme carried out for the European Space Agency (ESA) https://www.esa.int/Enabling_Support/Space_Engineering_Technology/SOLARIS/Cost_vs. _ben efits_studies
[8] https://www.caltech.edu/about/news/caltech-to-launch-space-solar-power-technology-demo- into-orbit-in-january

## Claims

1. A multi-sensor apparatus of modular construction for the detection of natural hydrogen and helium deposits, consisting of a casing tube, on the sides of which electrodes are placed and a protective disc, **characterised by the fact that** in the casing tube **(1)** there is a spacer **(4)** on which a gas sensor module **(5)** is placed, followed by a radon sensor module **(6),** a pumping module **(7),** a battery module **(8),** a logger **(9)** and a transmitter-receiver system located in the valve head **(10),** whereby the casing pipe **(1)** is provided with a groove **(11, 24, 30, 33, 39, 43)** inserted into the grooves **(11, 24, 30, 33, 39, 43)** and running inside the casing tube **(1)** through its entire length, inside of which is placed a probe measuring the pH of the soil, which protrudes outside the casing tube **(1).**

2. The apparatus according to claim 1, **characterised in that** the protective disc **(3)** is in the form of a metal mesh with a mesh size of < 50 micrometres.

3. The apparatus according to claims 1 or 2, **characterised in that** the spacer **(4)** has eight legs **(13)** which form, at the base of the spacer **(4),** eight chambers connected by an annular groove **(14)** intersecting each of the legs **(13),** each chamber containing a single ground gas inlet channel **(15)** which leads to a single sensor for detecting a specific type of gas, the sensor being located in a gas sensor module **(5),** furthermore in the groove **(14)** in one of the legs there is a water guide channel **(12).**

4. The apparatus according to claims 1-3, **characterised by** the fact that each inlet opening of the ground gas inlet channel **(15)** is surrounded by a socket **(16)** in which a protective ring with a metal mesh of less than 50 micrometres is mounted.

5. The apparatus according to claims 1-4, **characterised in that** in the central part of the spacer **(4)** there is a radon inlet channel **(18)** that runs inside the cylinder **(19)** projecting from the spacer **(4)** and which leads to the radon sensor located in the radon sensor module **(6),** the radon inlet channel inlet **(18)** being surrounded by a socket **(17)** in which a protective ring with a protective metal mesh is mounted while the cylinder **(19)** passes through the gas sensor module **(5)** and leads to the radon sensor module **(6).**

6. The apparatus according to claims 1 - 5, **characterised in that** in the upper part of the spacer **(4)** projects a ring **(20)** with an internal thread to which the sensor module is screwed gas **(5).**

7. The apparatus according to claims 1-5, **characterised in that** in the central part of the gas sensor module **(5)** there is a through circular opening **(21)** through which a cylinder **(19)** belonging to the spacer **(4)** passes and enters the radon sensor module **(6),** the gas sensor module **(5)** being internally divided into eight separate isolated chambers, in each of which a single sensor measuring the concentration of a gas selected from hydrogen, helium, nitrogen, CO₂, CH₄, H₂S is placed and the measurement data from each sensor is collected by an internal electronic circuit and transmitted to logger **(9)** via a bus.

8. The apparatus according to claim 7 **characterised by** the fact that a separate opening **(22)** leads to each of the eight chambers in the gas sensor module **(5),** which is an extension of the ground gas inlet channels **(15),** which are located in the spacer **(4)** and from each of the eight chambers in the gas sensor module **(5),** the analysed gas is discharged through a separate outlet channel **(25);** whereby a separate and isolated channel **(23)** also leads through the gas sensor module **(5),** through which water pumped from the bottom of the borehole flows and the channel **(23)** is an extension of the guide channel **(12)** in the spacer **(4)** and leads to the pumping module **(7).**

9. The apparatus according to claim 1, **characterised in that** there are channels **(27)** passing through the radon sensor module **(6)** to discharge gas from the chambers of the gas sensor module **(5)** to a pumping module **(7)** located above the radon sensor module **(6),** with an opening **(26)** in the lower part of the radon sensor **(6)** and an opening **(32)** in the upper part, with a radon sensor located in the radon sensor chamber **(6).**

10. The apparatus according to claim 9, **characterised in that** the radon sensor module **(6)** is provided with two rings: the upper ring **(31)** and the lower ring **(28),** which have female threads.

11. The apparatus according to claim 1, **characterised in that** in the lower part of the pumping module **(7)** there are openings **(35, 36, 34),** which supply respectively gases from the chambers of the gas sensor module **(5),** gas from the radon sensor module **(6),** water pumped from the bottom of the interior of the casing tube **(1),** moreover from the pumping module **(7)** there emerges a channel **(37)** while at the bottom there projects a threaded cylinder **(35),** which enables its connection with the earlier radon sensor module **(6).**

12. The apparatus according to claim 1, **characterised in that** the battery module **(8)** is connected to the downstream pumping module **(7)** and the upstream logger (9) respectively by means of rings **(41)** and **(38)** with male threads, furthermore along the battery module **(8)** runs a keyway **(39)** which is an extension of the keyways **(33, 30, 24, 11)** occurring in elements **(7, 6, 5, 4)** respectively.

13. The apparatus according to claim. 1 **characterised by** the fact that on the surface of the casing tube **(1) there** is a valve head with a transmitting and receiving system **(10)** in the form of a spherical bowl **(44),** which by means of a ring with an internal thread **(45)** is connected to the other modules **(4** - **9),** whereby on the spherical bowl there is an opening **(46)** through which water as well as ground gases are removed in the form of a spray; moreover, on the spherical bowl there are also LEDs **(47).**

14. A method for detecting deposits of natural hydrogen and/or helium using a multi-sensory modular device as described in claim 1 **characterised in that** a combination of hydrogen and helium and/or radon detection is used, the method comprising the following steps:
a) preselection of sites on the basis of publicly available geological data;
b) search for so-called 'fairy eyes' using optical or radar imagery from satellites, airborne or drones, and orotophotos may also be used;
c) installation of the equipment according to claim. 1 at the sites selected in steps a) and b) for a period of at least several months, in order to study the daily changes in the composition of the ground gas on the basis of data periodically transmitted from the devices to a central database via a mobile network or, in the absence thereof, via satellite; in addition, information on the location of the device is transmitted, the determination of which is made possible by an integrated GPS receiver;
d) mapping of environmental and weather conditions of equipment installation sites by checking the weather conditions in the area of installation of the respective sensor, data on soil moisture and pH, air temperature and pressure from sensors in the unit;
e) performing SAR Doppler tomography of a selected area;
f) performing a flight over the site selected in previous steps a) to e) by a drone or aircraft with a gravimetric sensor and a magnetic sensor installed;
g) identification of the likely location of natural hydrogen and/or helium deposits, where seismic geophysical surveys should be initiated and a test hole drilled.
